# EUROPEAN PATENT SPECIFICATION

(11) **EP 4 043 425 B1**
(45) Date of publication and mention of the grant of the patent: **30.04.2025**
(21) Application number: 20881557.1
(22) Date of filing: 29.10.2020
(51) Int. Cl.: C07C 29/94, C07C 29/143, C07C 35/06, B01J 23/46, B01J 37/08, C07C 45/59, C07C 49/597

(54) **METHOD FOR PREPARING 1,3-CYCLOPENTANEDIOL**
VERFAHREN ZUM HERSTELLEN VON 1,3-CYCLOPENTANDIOL
PROCÉDÉ DE PRÉPARATION DE 1,3-CYCLOPENTANEDIOL

(30) Priority: 29.10.2019 KR 20190135863
(43) Date of publication of application: 17.08.2022
(73) Proprietor: Kolon Industries, Inc., Seoul 07793 (KR)
(72) Inventor: JEONG, Mee Hye, Seoul 07793 (KR); YONG, Da Kyoung, Seoul 07793 (KR); HA, Ji Min, Seoul 07793 (KR); PARK, Ki Hyun, Seoul 07793 (KR)
(74) Representative: Ter Meer Steinmeister & Partner
(86) International application number: PCT/KR2020/014970
(87) International publication number: WO 2021/086089

(56) References cited:
- CN-A- 106 866 331
- CN-A- 106 866 345
- CN-A- 106 866 364
- JP-A- 2015 024 969
- LI GUANGYI, LI NING, ZHENG MINGYUAN, LI SHANSHAN, WANG AIQIN, CONG YU, WANG XIAODONG, ZHANG TAO: "Industrially scalable and cost-effective synthesis of 1,3-cyclopentanediol with furfuryl alcohol from lignocellulose", GREEN CHEMISTRY, vol. 18, no. 12, 14 March 2014 (2014-03-14), pages 3607 - 3613, XP055787391, DOI: 10.1039/C6GC00341A

## Description

### [Technical Field]

The present invention relates to a method for preparing 1,3-cyclopentanediol.

### [Background Art]

In general, polymers such as polycarbonates, polyesters, and the like are prepared by using raw materials derived from petroleum resources. However, in recent years, since there are concerns about depletion of the petroleum resources, polymer resins need to be provided by using raw materials obtained from biomass resources such as plants and the like. In addition, since global warming due to an increase and accumulation of carbon dioxide emissions causes climate change, etc., development of polymers by using raw materials of monomers derived from plants emitting very little carbon during the manufacturing process is required.

1,3-cyclopentanediol is synthesized from cyclopentadiene, and the cyclopentadiene may be obtained by cracking dicyclopentadiene, which is shown in Reaction Scheme 1.

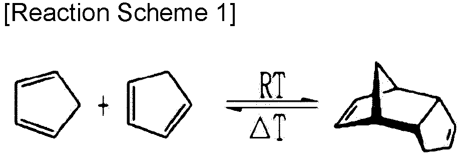

The cyclopentadiene is highly reactive in the Diels-Alder reaction and converted back to the dicyclopentadiene within just a few hours at room temperature. Accordingly, the cyclopentadiene has a drawback of being stored at -20 °C just for a couple of days use.

Furfural is an organic compound extracted from plant materials such as corn cobs, oats, bran, rice bran, bagasse, and sawdust. In other words, the furfural is a biomass-based chemical material from which the 1,3-cyclopentanediol may be prepared.

The 1,3-cyclopentanediol is prepared from the biomass-derived furfural in three steps by Reaction Scheme 2.

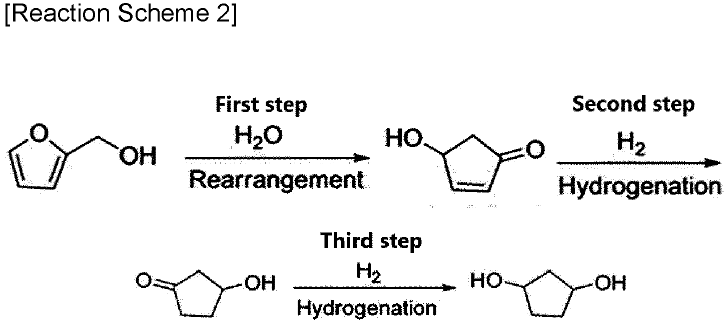

The reaction product of the 2^{nd} step, 4-hydroxy-2-cyclopentenone, is hydrogenated to form an intermediate 3-hydroxycyclopentanone, and the 3-hydroxycyclopentanone may form 1,3-cyclopentanediol by a hydrogenation reaction.

However, the conventional method of preparing 1,3-cyclopentanediol uses different types of catalysts in the 2^{nd} and 3^{rd} steps, which brings about a problem of complicated reaction conditions and long reaction time. In addition, there is another problem of significantly lowering a yield of the 1,3-cyclopentanediol due to the formation of the intermediate. CN 106 866 364 A relates to a process for the preparation of 1,3-cyclopentanediol from furfuryl alcohol.

Accordingly, a new method of preparing the 1,3-cyclopentanediol, which solves the problems, is required.

### [Prior Art]

### [Patent Document]

Korean Patent Laid-Open Publication No. 10-2015-0067788

### [Disclosure]

### [Technical Problem]

In order to solve the problems, the present inventors have conducted various studies and discovered a method of directly forming 1,3-cyclopentanediol without forming an intermediate of 3-hydroxycyclopentanone by adding a ruthenium catalyst from which a surface oxide layer is removed, to 4-hydroxy-2-cyclopentenone, which may simplify the preparing process, shorten reaction time, and increase a yield of the 1,3-cyclopentanediol.

Accordingly, an object of the present invention is to provide a method for preparing 1,3-cyclopentanediol, in which 1,3-cyclopentanediol can be prepared directly from 4-hydroxy-2-cyclopentenone without forming an intermediate.

### [Technical Solution]

In order to achieve the above object, the present invention provides a method for preparing 1,3-cyclopentanediol including adding a catalyst to 4-hydroxy-2-cyclopentenone to produce 1,3-cyclopentanediol, wherein the catalyst is a ruthenium catalyst from which the surface oxide layer is removed.

In the preparing of 1,3-cyclopentanediol, 3-hydroxycyclopentanone, which is a reaction intermediate, is not produced.

The ruthenium catalyst from which the surface oxide layer is removed is prepared by heat-treating ruthenium.

The ruthenium catalyst from which the surface oxide layer is removed is included in an amount of 0.05 parts by weight to 0.3 parts by weight based on 100 parts by weight of 4-hydroxy-2-cyclopentenone.

The preparing of 1,3-cyclopentanediol may be performed at a temperature of 70 °C to 200 °C and a pressure of 30 bar to 70 bar in a hydrogen atmosphere.

The preparing of 1,3-cyclopentanediol may be performed for 30 minutes to 2 hours.

The method may further include removing moisture from the 4-hydroxy-2-cyclopentenone before adding the catalyst to the 4-hydroxy-2-cyclopentenone.

The 4-hydroxy-2-cyclopentenone may be prepared from an aqueous solution of furfuryl alcohol.

The furfuryl alcohol may be derived from a biomass.

In addition, the present invention provides 1,3-cyclopentanediol prepared by the above method.

### [Advantageous Effects]

In the method for preparing 1,3-cyclopentanediol of the present invention, the intermediate 3-hydroxycyclopentanone is not formed during the preparing process, and thus the preparing steps may be reduced, thereby simplifying the preparing process and shortening the reaction time. In addition, the yield of 1,3-cyclopentanediol may be increased.

### [Description of the Drawings]

FIG. 1 is a graph showing GC-FID results of Examples 1 to 4.
FIG. 2 is a graph of GC-MASS results of Example 5.
FIG. 3 is a graph showing GC-FID results of Comparative Examples 1 to 3 and 6.
FIG. 4 is a graph showing GC-FID results of Comparative Examples 4 and 5.

### [Mode for Invention]

Hereinafter, the present invention will be described in more detail.

A conventional method for preparing 1,3-cyclopentanediol includes (1) adding a catalyst to 4-hydroxy-2-cyclopentenone to produce 3-hydroxycyclopentanone, and (2) adding a catalyst to the 3-hydroxycyclopentanone to produce 1,3-cyclopentanediol.

This is shown in Reaction Scheme 3.

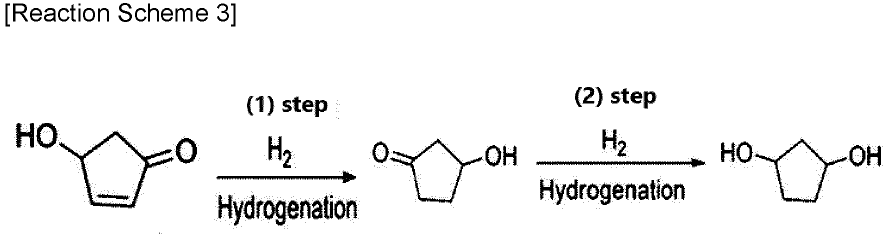

The 3-hydroxycyclopentanone is an intermediate for forming the 1,3-cyclopentanediol, and the conventional method of preparing the 1,3-cyclopentanediol should go through two steps due to formation of the intermediate, which makes the process complicated, and specifically, different types of catalysts in the 1^{st} and 2^{nd} steps make the process complicated, and there is also a problem of long reaction time of 3 hours or more. In addition, there is another problem that a yield of the 1,3-cyclopentanediol is less than 40 % which is very low.

Accordingly, in order to solve the above problems, the present invention provides a method of directly preparing the 1,3-cyclopentanediol from 4-hydroxy-2-cyclopentenone without forming an intermediate of 3-hydroxycyclopentanone, which may simplify the preparation process, shorten reaction time, and increase a yield of the 1,3-cyclopentanediol.

In other words, the present invention provides the method of preparing the 1,3-cyclopentanediol by adding a catalyst to 4-hydroxy-2-cyclopentenone to prepare the 1,3-cyclopentanediol, wherein the catalyst is a ruthenium catalyst from which a surface oxide layer is removed.

Preparation of 1,3-cyclopentanediol of the present invention is shown in Reaction Scheme 4.

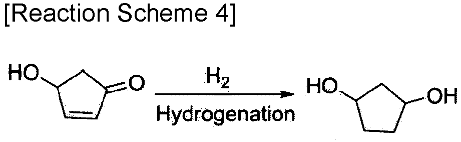

In other words, the present invention uses the ruthenium catalyst from which a surface oxide layer is removed and thus may directly prepare the 1,3-cyclopentanediol from 4-hydroxy-2-cyclopentenone without forming an intermediate of 3-hydroxycyclopentanone.

The ruthenium catalyst from which a surface oxide layer is removed refers to pure ruthenium from which the oxide layer is removed from the surface.

In general, there is a problem that an oxide layer (native oxide layer) surrounds ruthenium and thus inhibits a reaction. Accordingly, the present invention heat-treats a ruthenium catalyst to remove the oxide layer therearound and thus uses pure ruthenium from which the surface oxide layer is removed as a catalyst.

The heat treatment may be performed at 200 °C or more for 2 hours or more but is not limited thereto.

When the ruthenium catalyst from which the surface oxide layer is removed through the heat treatment is added, hydrogen gas (H₂, H-H group) and an alkene hydrocarbon with a double bond are formed on the ruthenium surface during the formation of the 1,3-cyclopentanediol. The hydrogen formed on the ruthenium surface reacts with the alkene hydrocarbon and converts it into a saturated hydrocarbon.

The process of producing the saturated hydrocarbon on the surface of the ruthenium catalyst from which the surface oxide layer is removed and the process of preparing the 1,3-cyclopentanediol from the 4-hydroxy-2-cyclopentenone simultaneously occur. Accordingly, the 1,3-cyclopentanediol may be prepared in one step without forming the intermediate of 3-hydroxycyclopentanone.

When a ruthenium catalyst from which a surface oxide layer is not removed is used during preparation of the 1,3-cyclopentanediol, since the oxide layer around the catalyst inhibits a reaction and thus forms an intermediate, a yield of the 1,3-cyclopentanediol is less than 40 %, but when the ruthenium catalyst from which the surface oxide layer is removed is used, since the intermediate is not formed, the yield of the 1,3-cyclopentanediol may be increased to 45 % or more.

The number of carbon atoms of the saturated hydrocarbon and the alkene hydrocarbon may be 2 to 10, and desirably 2 to 5, but is not limited thereto.

The ruthenium catalyst from which the surface oxide layer is removed is added to a solution in which the 4-hydroxy-2-cyclopentenone is dissolved in an organic solvent, and the organic solvent may include one or more selected from 2-methyltetrahydrofuran, methanol, and acetone, and desirably 2-methyltetrahydrofuran.

The ruthenium catalyst from which the surface oxide layer is removed is included in an amount of 0.05 to 0.3 parts by weight, and desirably 0.1 to 0.2 parts by weight, based on 100 parts by weight of 4-hydroxy-2-cyclopentenone.

When the ruthenium catalyst from which the surface oxide layer is removed is included in an amount of less than 0.05 parts by weight, since the double bond of the alkene hydrocarbon does not react and thus forms no saturated hydrocarbon, but when included in an amount of 0.3 parts by weight, since there are many unreacted catalysts, causing an economical loss, a ring opening phenomenon of cyclopentane due to an overreaction, or a side reaction may undesirably occur.

Since the 1,3-cyclopentanediol is prepared by a hydrogenation reaction of the 4-hydroxy-2-cyclopentenone, the 1,3-cyclopentanediol may be prepared under a hydrogen (H₂) atmosphere.

In addition, the reaction may be performed at a temperature of 70 °C to 200 °C under a pressure of 30 bar to 70 bar under the hydrogen atmosphere for 30 minutes to 2 hours, and desirably, at 100 °C to 150 °C under a pressure of 30 bar to 50 bar for 30 minutes to 1 hour.

When the temperature is less than 70 °C, the reaction may not occur, and when the temperature is greater than 200 °C, the ring opening phenomenon or side reaction of cyclopentane may occur. In addition, when the pressure is less than 30 bar, or the reaction time is less than 30 minutes, the reaction may not occur due to an insufficient H-H group on the ruthenium surface, and when the pressure is greater than 70 bar, the ring opening phenomenon of cyclopentane may occur. In addition, when the reaction time is greater than 2 hours, the reaction may proceed no more, which is not economical.

In addition, the present invention may further include removing moisture from the 4-hydroxy-2-cyclopentenone before adding the ruthenium catalyst, from which the surface oxide layer has been removed, to the 4-hydroxy-2-cyclopentenone.

The moisture may be removed by adding MgSO₄ to 4-hydroxy-2-cyclopentenone.

As the moisture is removed, when the 1,3-cyclopentanediol as a monomer is used to polymerize a polymer, an effect of controlling a polymerization rate of the polymer may be obtained.

The method of preparing 1,3-cyclopentanediol according to the present invention may accompany a side reaction, and in the side reaction, cyclopentanone or cyclopentanol may be formed, but these side reaction products are separately formed from an intermediate.

The 4-hydroxy-2-cyclopentenone is prepared from an aqueous solution of furfuryl alcohol, and the furfuryl alcohol is a biomass-derived compound.

The furfuryl alcohol is a derivative of furfural extracted from plant materials such as corn cobs, oats, bran, rice bran, and sawdust.

Specifically, the 4-hydroxy-2-cyclopentenone may be prepared by adding a catalyst to an aqueous solution of biomass-derived furfuryl alcohol.

The catalyst may include at least one selected from calcium oxide, lead oxide, aluminum oxide, iron oxide, calcium chloride, zinc acetate, para-toluene sulfonic acid, stannous chloride, stannous sulfate, stannous oxide, tin oxide, stannous octoate, tetraphenyl tin, a tin powder, and titanium tetrachloride, and desirably calcium oxide.

In addition, the catalyst may be included in an amount of 0.1 to 10 parts by weight, desirably 0.1 to 5 parts by weight, based on 100 parts by weight of the aqueous solution of furfuryl alcohol. When the catalyst is included in an amount of less than 0.1 parts by weight, the reaction may not occur, preparing no 4-hydroxy-2-cyclopentenone, and when the catalyst is included in an amount of greater than 10 parts by weight, there are many unreacted catalysts, which is not economical.

In addition, the 4-hydroxy-2-cyclopentenone may be prepared by adding a catalyst to an aqueous solution of furfuryl alcohol and performing a reaction at 100 °C to 300 °C for 10 minutes to 1 hour.

That is, the process for preparing 1,3-cyclopentanediol from the biomass-derived furfuryl alcohol is shown in Reaction Scheme 5.

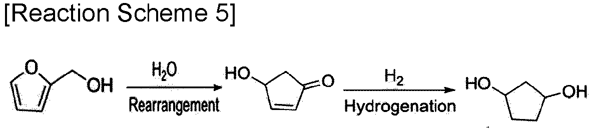

Accordingly, the present invention uses a ruthenium catalyst from which a surface oxide layer is removed and thus directly prepares 1,3-cyclopentanediol from 4-hydroxy-2-cyclopentanone without forming an intermediate of 3-hydroxycyclopentanone, which may provide a simpler process, shorten reaction time, and increase a yield of the 1,3-cyclopentanediol.

In addition, the 1,3-cyclopentanediol may be prepared from furfuryl alcohol derived from a biomass, which may achieve environmentally-friendly and economical effects.

In addition, the present invention relates to the 1,3-cyclopentanediol prepared in the method of the present invention.

### DETAILED DESCRIPTION OF THE EMBODIMENTS

Hereinafter, although described with reference to examples of the present invention, the following examples are for illustrating the present invention, and the scope of the present invention is not limited thereto.

### < Preparation of 1,3-Cyclopentanediol>

Examples 1 to 5 and Comparative Examples 1 to 6.

An aqueous solution was prepared by dissolving 14 g of furfuryl alcohol, a derivative of furfural extracted from a biomass, in 685.97 mL of water. Subsequently, 0.028 g of a calcium oxide catalyst was added to the aqueous solution and then stirred at 200 °C for 0.5 hours, preparing 4-hydroxy-2-cyclopentenone.

Then, 248 g of the 4-hydroxy-2-cyclopentenone was dissolved in 2000 mL of 2-methyltetrahydrofuran, preparing a solution. Then, a catalyst was added thereto to perform a hydrogenation reaction under the following conditions, preparing 1,3-cyclopentanediol (CPdiol) derived from the biomass.

Table 1 shows a type, a reaction temperature, a reaction pressure, and reaction time of each catalyst of Examples 1 to 5 and Comparative Examples 1 to 6.

**(Table 1)**

| | Type of catalyst | Content of catalyst (parts by weight) | Reaction temperature (°C) | Reaction pressure (bar) | Reaction time (h) |
|---|---|---|---|---|---|
| Example 1 | Ru/C(R) | 0.2 | 100 | 50 | 1 |
| Example 2 | Ru/C(R) | 0.2 | 100 | 50 | 2 |
| Example 3 | Ru/C(R) | 0.4 | 100 | 50 | 0.5 |
| Example 4 | Ru/C(R) | 0.4 | 100 | 50 | 1 |
| Example 5 | Ru/C(R) | 0.4 | 100 | 50 | 1 |
| Comparative Example 1 | Ru/C | 0.1 | 100 | 30 | 2 |
| Comparative Example 2 | Ru/C | 0.1 | 150 | 30 | 2 |
| Comparative Example 3 | Ru/C | 0.1 | 100 | 50 | 2 |
| Comparative Example 4 | Ru/C | 0.1 | 100 | 50 | 1 |
| Comparative Example 5 | Ru/C | 0.2 | 100 | 50 | 1 |
| Comparative Example 6 | Pd/C | 0.1 | 150 | 30 | 2 |

The Ru/C was a ruthenium catalyst from which a surface oxide layer was not removed, the Ru/C(R) was a ruthenium catalyst from which the surface oxide layer was removed, and the Pd/C was a palladium catalyst from which the surface oxide layer was not removed. The ruthenium catalyst from which the surface oxide layer was removed (CEM Corp.) was obtained by heat-treating ruthenium at a temperature of 280 °C or higher for 3 hours or more to remove the surface oxide layer.

In addition, the contents of the catalysts were respectively based on 100 parts by weight of 4-hydroxy-2-cyclopentenone.

In addition, in Example 5, a reaction was performed under the following reaction conditions by adding the ruthenium catalyst from which the surface oxide layer was removed, after removing moisture from the 4-hydroxy-2-cyclopentenone, wherein the moisture removal was performed by using MgSO₄.

### Experimental Example 1

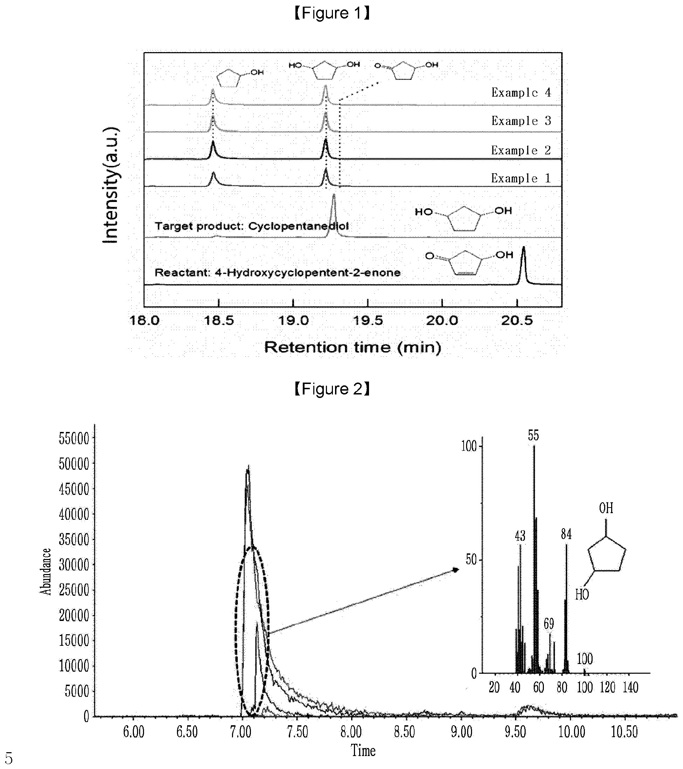

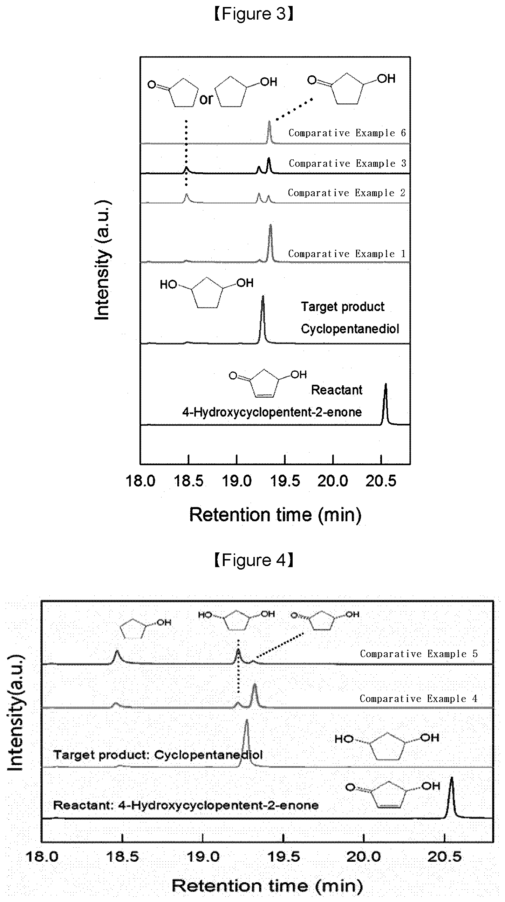

The 1,3-cyclopentanediols according to Examples 1 to 5 and Comparative Examples 1 to 6 and the intermediate of 3-hydroxycyclopentanone were measured with respect to yields by using GC-FID (Agilent 7890B GC / 5975C MSD), and the results are shown in FIGS. 1 to 4. FIG. 2 is a graph showing the GC-MASS result of the 1,3-cyclopentanediol according to Example 5. Reaction conditions of GC-FID are as follows, and the yields of the 1,3-cyclopentanediol and the intermediate of 3-hydroxycyclopentanone are summarized in Table 2.

### <Reaction Condition of GC-FID>

Injector condition: 250 °C, 4 µL, Split = 20:1
Column: HP-INNOWAX (30 m x 0.32 mm x 0.25 um)
Pressure rate: 2.33 mL/min
Mobile phase: He, 1 mL/min
Oven temperature: From 80 °C, 10 °C/min to 320 °C, hold 5 min
Detector: 28 to 600 m/z scan
Injection method (Sampling): Direct injection

**(Table 2)**

| | Type of catalyst | CPdiol yield | Intermediate yield | CPone yield | CPol yield |
|---|---|---|---|---|---|
| Example 1 | Ru/C(R) | 47.7 % | 0 % | 0 % | 52.3 % |
| Example 2 | Ru/C(R) | 48.6 % | 0 % | 0 % | 51.4 % |
| Example 3 | Ru/C(R) | 49.7 % | 0 % | 0 % | 50.3 % |
| Example 4 | Ru/C(R) | 50.7 % | 0 % | 0 % | 49.3 % |
| Example 5 | Ru/C(R) | 53.9 % | 0 % | 0 % | 46.1 % |
| Comparative Example 1 | Ru/C | 4.6 % | 90.5 % | 0 % | 4.9 % |
| Comparative Example 2 | Ru/C | 28.3 % | 21 % | 11.2 % | 39.5 % |
| Comparative Example 3 | Ru/C | 22.5 % | 47.7 % | 0 % | 29.9 % |
| Comparative Example 4 | Ru/C | 15 % | 64.1 % | 0 % | 20.9 % |
| Comparative Example 5 | Ru/C | 39.1 % | 9.2 % | 0 % | 51.7 % |
| Comparative Example 6 | Pd/C | 0.7 % | 97.8 % | 1.6 % | 0 % |

In Table 2, CPone and CPol respectively indicate side reaction products of cyclopentanone and cyclopentenol.

Referring to the results of Table 2, the methods of Examples 1 to 5 using the ruthenium catalyst (Ru/C(R)) from which the surface oxide layer was removed formed no intermediate of 3-hydroxycyclopentanone but exhibited a yield of 1,3-cyclopentanediol (CPdiol) of 45 % or more. In particular, Example 5, in which the reaction was performed under the above conditions by removing moisture from 4-hydroxy-2-cyclopentenone and adding the ruthenium catalyst from which the surface oxide layer was removed, exhibited the highest yield of 1,3-cyclopentanediol.

On the contrary, the methods of Comparative Examples 1 to 5 using the ruthenium catalyst (Ru/C) from which the surface oxide layer was not removed exhibited a very low yield of 1,3-cyclopentanediol and formation of an intermediate. In addition, the lower the yield of the 1,3-cyclopentanediol, the higher the yield of the intermediate.

Accordingly, the method of preparing 1,3-cyclopentanediol according to the present invention formed 1,3-cyclopentanediol without forming an intermediate of 3-hydroxycyclopentanone by using a ruthenium catalyst from which the surface oxide layer was removed, and accordingly, provided a simpler process and improved a yield thereof.

### [Industrial Applicability]

The present invention relates to a method of preparing 1,3-cyclopentanediol, wherein an intermediate of 3-hydroxycyclopentanone is not formed, and may reduce steps of preparing the 1,3-cyclopentanediol and has an effect of simplifying the preparation process and shortening reaction time. In addition, there is an effect of increasing a yield of the 1,3-cyclopentanediol.

## Claims

1. A method of preparing 1,3-cyclopentanediol, comprising
adding a catalyst to 4-hydroxy-2-cyclopentenone to produce 1,3-cyclopentanediol,
wherein the catalyst is a ruthenium catalyst from which the surface oxide layer is removed,
wherein the ruthenium catalyst from which the surface oxide layer is removed is prepared by heat-treating ruthenium, and
wherein the ruthenium catalyst from which the surface oxide layer is removed is included in an amount of 0.05 to 0.3 parts by weight based on 100 parts by weight of 4-hydroxy-2-cyclopentenone.

2. The method of claim 1, wherein in the preparing of 1,3-cyclopentanediol, 3-hydroxycyclopentanone, which is a reaction intermediate, is not produced.

3. The method of claim 1, wherein the preparing of 1,3-cyclopentanediol is performed at a temperature of 70 to 200 °C and a pressure of 30 to 70 bar in a hydrogen atmosphere.

4. The method of claim 1, wherein the preparing of 1,3-cyclopentanediol is performed for 30 minutes to 2 hours.

5. The method of claim 1, which further comprises removing moisture from the 4-hydroxy-2-cyclopentenone before adding the catalyst to the 4-hydroxy-2-cyclopentenone.

6. The method of claim 1, wherein the 4-hydroxy-2-cyclopentenone is prepared from an aqueous solution of furfuryl alcohol.

7. The method of claim 6, wherein the furfuryl alcohol is derived from a biomass.

## Patentansprüche

1. Verfahren zur Herstellung von 1,3-Cyclopentandiol, umfassend
Zugeben eines Katalysators zu 4-Hydroxy-2-cyclopentenon, um 1,3-Cyclopentandiol herzustellen,
wobei der Katalysator ein Rutheniumkatalysator ist, von dem die Oberflächenoxidschicht entfernt wird,
wobei der Rutheniumkatalysator, von dem die Oberflächenoxidschicht entfernt wird, durch Wärmebehandlung von Ruthenium hergestellt wird, und
wobei der Rutheniumkatalysator, von dem die Oberflächenoxidschicht entfernt wird, in einer Menge von 0,05 bis 0,3 Gewichtsteilen, bezogen auf 100 Gewichtsteile 4-Hydroxy-2-cyclopentenon, enthalten ist.

2. Verfahren nach Anspruch 1, wobei beim Herstellen von 1,3-Cyclopentandiol 3-Hydroxycyclopentanon, welches ein Reaktionszwischenprodukt ist, nicht hergestellt wird.

3. Verfahren nach Anspruch 1, wobei das Herstellen von 1,3-Cyclopentandiol bei einer Temperatur von 70 bis 200°C und einem Druck von 30 bis 70 bar in einer Wasserstoffatmosphäre durchgeführt wird.

4. Verfahren nach Anspruch 1, wobei das Herstellen von 1,3-Cyclopentandiol für 30 Minuten bis 2 Stunden durchgeführt wird.

5. Verfahren nach Anspruch 1, welches ferner das Entfernen von Feuchtigkeit aus dem 4-Hydroxy-2-cyclopentenon vor dem Zugeben des Katalysators zu dem 4-Hydroxy-2-cyclopentenon umfasst.

6. Verfahren nach Anspruch 1, wobei das 4-Hydroxy-2-cyclopentenon aus einer wässrigen Lösung von Furfurylalkohol hergestellt wird.

7. Verfahren nach Anspruch 6, wobei der Furfurylalkohol aus einer Biomasse gewonnen wird.

## Revendications

1. Procédé de préparation de 1,3-cyclopentanediol, comportant l'étape consistant à :
ajouter un catalyseur à du 4-hydroxy-2-cyclopenténone pour produire du 1,3-cyclopentanediol,
dans lequel le catalyseur est un catalyseur au ruthénium duquel la couche d'oxyde superficielle est retirée,
dans lequel le catalyseur au ruthénium duquel la couche d'oxyde superficielle est retirée, est préparé par un traitement thermique du ruthénium, et
dans lequel le catalyseur au ruthénium duquel la couche d'oxyde superficielle est retirée, est inclus en une quantité de 0,05 à 0,3 partie en poids sur la base de 100 parties en poids de 4-hydroxy-2-cyclopenténone.

2. Procédé selon la revendication 1, dans lequel pendant la préparation de 1,3-cyclopentanediol, du 3-hydroxycyclopentanone, qui est un produit intermédiaire de réaction, n'est pas produit.

3. Procédé selon la revendication 1, dans lequel la préparation du 1,3-cyclopentanediol est réalisée à une température de 70 °C à 200 °C et une pression de 30 bar à 70 bar dans une atmosphère d'hydrogène.

4. Procédé selon la revendication 1, dans lequel la préparation du 1,3-cyclopentanediol est réalisée pendant 30 secondes à 2 heures.

5. Procédé selon la revendication 1, qui comporte en outre d'éliminer l'humidité du 4-hydroxy-2-cyclopenténone avant d'ajouter le catalyseur au 4-hydroxy-2-cyclopenténone.

6. Procédé selon la revendication 1, dans lequel le 4-hydroxy-2-cyclopentènone est préparé à partir d'une solution aqueuse d'alcool furfurylique.

7. Procédé selon la revendication 6, dans lequel l'alcool furfurylique est dérivé d'une biomasse.
